# EUROPEAN PATENT SPECIFICATION

(11) **EP 1 779 809 B1**
(45) Date of publication and mention of the grant of the patent: **11.08.2010**
(21) Application number: 05757084.8
(22) Date of filing: 08.06.2005
(51) Int. Cl.: A61F 2/06, A61M 29/00

(54) **COMBINED FILM-COATED STENT WHICH CAN BEND IN ANY DIRECTION**
KOMBINIERTER FILMBESCHICHTETER STENT, DER IN JEDER RICHTUNG BIEGBAR IST
PATE A MOULAGE SILICONEE A REVETEMENT PELLICULAIRE COMBINE POUVANT ETRE PLIEE DANS TOUTES LES DIRECTIONS

(30) Priority: 17.08.2004 CN 200410053816
(43) Date of publication of application: 02.05.2007
(73) Proprietor: Microport Medical (Shanghai) Co., Ltd., 201203 Shanghai (CN)
(72) Inventor: Luo, Qiyi, Pudong New District, Shanghai 201203 (CN); Xu, Shangdong, Pudong New District, Shanghai 201203 (CN); Nie, Honglin, Pudong New District, Shanghai 201203 (CN)
(74) Representative: Graf Glück Habersack Kritzenberger
(86) International application number: PCT/CN2005/000815
(87) International publication number: WO 2006/017968

(56) References cited:
- EP-A1- 1 262 153
- WO-A-01/01887
- CN-Y- 2 527 242
- CN-Y- 2 582 641
- US-A1- 2002 029 077
- US-A1- 2002 133 244
- US-A1- 2003 139 797
- US-A1- 2003 195 614

## Description

### Abstract

A multi-unit stent-graft which can bend in any direction comprises multiple stent-graft units stacked upon each other and sutured in adjacent turns with the proximal stent-graft unit overlapping partly the neighboring one to combine to form a tubular stent without a connector bar in the place where flexing required. Each stent-graft unit comprises a stent member sutured and coupled to a tubular graft member. The stent member is formed from a single continuous wire arranged in a tubular configuration with multiple bends and having multiple undulations with each undulation having an apex.

The invention described below relate the field of a mini-invasive medical devices for use in treatment of vascular diseases. More specifically, the invention described below relate to a stent-grafts which can bend in all directions.

Aneurysms are localized, pathological dilatations of a blood vessel caused by a disease or weakening of the vessel's wall which are very dangerous to the patients. Aneurysms occur more often in people over the age of fifty, but can occur in people of all age groups. Referred to some articles, if without timely treatment, the death rate of an aneurysm patient can be 15% within 24 hours, reach 30% within a week, and up to 75% within a month. Only 17% of the patients without any treatment can keep alive after 5 years. Thus the patients diagnosed as aneurysms should be treated timely with proper procedures.

Mini-invasive treatments which employ the endovascular isolating method are widely used this years. It results from advances in the catheter-based technologies, using a delivery system with a small catheter to deliver the compressed stent-graft to the diseased vessel and release the stent-graft to cover the aneurysm that isolate the aneurysm bubble from the blood flow and pressure. The blood in the space formed by the stent-graft and the aneurysm bubble turn into thrombus steadily and absorbed eventually by the vessel tissue thus eliminate the danger of the aneurysms.

A wide range of endovascular stent-grafts have been developed that are adapted for implantation within a body lumen. Most of the present stent-grafts contain a longitudinal connecting bar connecting stent members disposed on the distal end of the stent-graft to stent members disposed on the distal end of the stent-graft to maintain the total length of the stent-graft.

While maintaining the total length of the stent-graft, use of a connector bar adversely affects flexibility of stent-grafts along the longitudinal axis. When a stent-graft having a connector bar is disposed in a curved artery, such as the aortic arch, the connector bar must be disposed longitudinally along the outside curve of the arch of the aorta. Otherwise, if the connector bar is not disposed along the outside curve of the arch, but instead is disposed along the inside curve of the arch, the stent-graft can kink or buckle causing: a rough inner lumen to form in the stent-graft, a decrease in the contact surface between the graft and blood vessel, a decrease in the fixing performance of the stent-graft, higher blood flush force on the stent-graft and an increase in potential for stent-graft migration which can lead to injury or patient death. Thus, while designing and making a stent-graft with a connecting bar, designers should take many potential risks into account to make sure that the stent-graft be delivered and released in the vessel in a right direction. This adds to the difficulties in designing and making, and procedures as wall, while some risks still remain. What is needed is a stent-graft without a connector bar for use in a curved artery where flexing is required for placement.

US 2003/0195614 A1 discloses a modular endoluminal stent-graft with stent-graft units that are assembled inside the vessel during surgery to form a branched prosthesis.

Further, WO 01/01887 A1 describes an implantable composite intraluminal prosthesis comprising a substantially continuous polytetrafluoroethylen tubular inner body; a longitudinally non-continuous outer tubular body; and a circumferentially distensible support structure interposed between the inner and outer tubular bodies, said outer tubular body being formed of polytetrafluoroethylen components, having a longitudinal length and a width, said longitudinal length being greater than said width, said components completely overlying the dispensable support structure, whereby axial and circumferential compliance is provided to said prosthesis.

### Summary

A multi-unit stent-graft is disclosed for use of treatment of aortic aneurysm diseases. The multi-unit stent-graft presents a stable construction, smooth inner lumen while flexing and can bend in all directions which get rid of the limitation of present products.

The present invention provides a tubular endoluminal prosthesis as recited in claim1.

The invention discloses a solution of former potential risks as follows.
Said multi-unit stent-graft disclosed comprises multiple stent-graft units stacked upon each other and sutured in adjacent turns with the proximal stent-graft unit overlapping partly the neighboring one to combine to form a tubular stent-graft without a connector bar in the place where flexing required. Each stent-graft unit comprises a stent member coupled to a graft member made of biocompatible material defining a tube. The multi-unit stent-graft may further comprise one or more open stent members.

Here the proximal end indicates the end portion in the stent-graft which is closer to the heart, and the distal end indicates the one which is away from the heart.

In each stent-graft unit, the stent member could either be disposed and sutured within the inner diameter or about the outer diameter of the graft member.

The distal most stent-graft unit comprises a graft member with a first end portion and a second end portion slightly exceeding the length of the stent member. The other stent-graft units, also called proximal stent-graft units, have a graft member with the first end portion slightly exceeding the length of the stent member and the second end portion exceeding the length of the stent member to a greater extent creating an overlapping length in the stent-graft. The axial length of the graft member of the distal most stent-graft unit ranges from 10 to 40 mm, and the axial length of the graft member of other stent-graft units range from 14 to 60 mm. The number of turns of waves of said stent member is from 4 to 10. The height between the peak and the bottom of waving ranges from 10 to 40 mm.

The stent member may be made of any suitable material, such as nitinol (in either super-elastic or shape memory form), CoCr alloy, or 316L/317L stainless steel. The wire OD ranges from 0.254 to 0.635 mm. The tubular graft members may manufactured from polytetrafluoroethylene (PTFE), expanded polytetrafluoroethylene (PTFE), polyester fabric, polyamide fabric, polypropylene, or other suitable biocompatible materials. The suture thread may be made of any suitable material, such as polypropylene, polyester, polyamide, silk, polytetrafluoroethylene (PTFE), polyfluoroethylene, or other suitable biocompatible materials.

The stent member is firstly formed from a single continuous wire arranged in a complanate configuration with multiple bends and having multiple undulations with each undulation having an apex by cold deformation, or heat treatment, or combination of cold deformation and heat treatment, and then has two ends of the wire joined together by welding or mechanical crimping using a alloy tube to define a tubular configuration. The alloy tube may be made of any suitable material, such as nitinol, CoCr alloy, or 316L/317L stainless steel.

Comparing to current stent-grafts sold in market, the advantages of the present invention are as follows:
As the stent-graft disclosed in this invention uses the graft member itself as connecting material, instead of using a connecting bar to connect the distal most stent member and the proximal most stent member, the stent-graft can bend in any direction. During the designing, manufacturing and using, no attention to the direction is necessary, which would reduce the production and quality control cost and simplify the operation of the device during the surgery. Without using the connecting bar, it eliminates all the potential risks due to the directional property of current sold stent-grafts. The invention disclosed can also provides a smooth inner surface after placement in an curved inner lumen of human body, thus decreases the affections to the hydromechanical state within the vessel, lowers the flushing force of blood flow on the stent-graft and the risk of stent-graft migration, it will also decrease the formation of thrombus.

### Brief Description of the Drawings

Figure 1 is an embodiment of current invention that illustrates a stent-graft.
Figure 2 is another embodiment of current invention that illustrates a stent-graft.
Figure 3 illustrates the configuration of the proximal stent-graft unit.
Figure 4 illustrates the connecting configuration of neighbouring stent-graft units.
Figure 5 illustrates the complanate configuration of the stent member.
Figure 6 illustrates the performance of the multi-unit stent-graft while bending.

### Detailed description of the invention

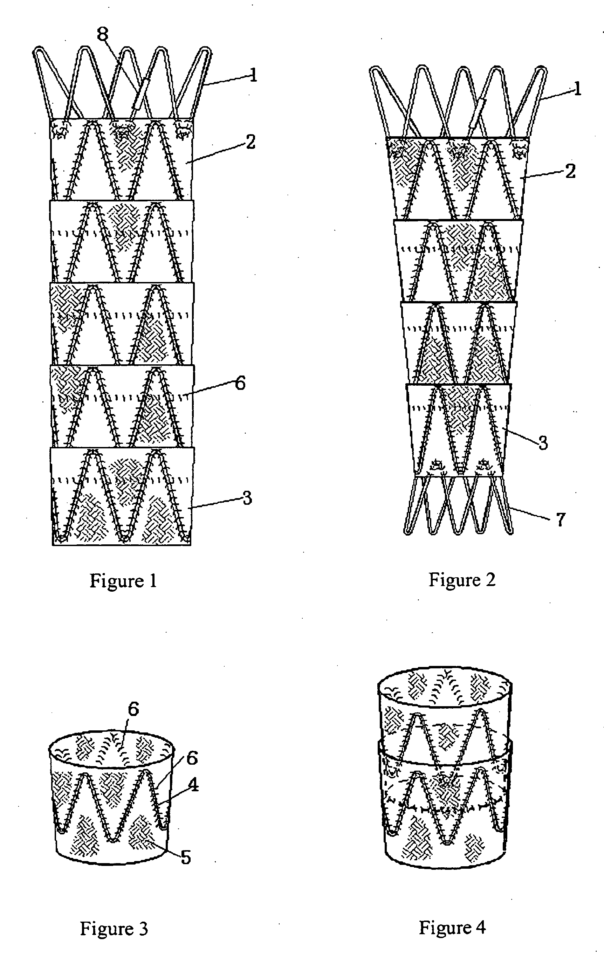

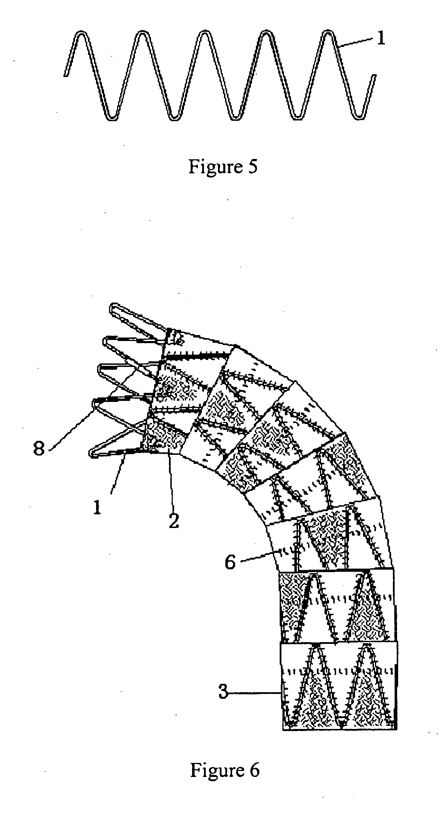

Referring to the figures 1, 2, 5 and 6, the multi-unit stent-graft which can bend in any direction disclosed in the invention comprises multiple proximal stent-graft units 2 and a distal most stent-graft unit 3 stacked upon each other and sutured in adjacent turns with the proximal stent-graft unit overlapping partly the neighboring one to combine to form a tubular stent-graft. Each stent-graft unit comprises a stent member 4 coupled and sutured with a tubular graft member 5 by the suture thread 6. Said stent member is formed from a single continuous wire arranged in a tubular configuration with multiple bends and having multiple undulations with each undulation having an apex.

Referring to the figure 2, the multi-unit stent-graft may optionally comprises a proximal open stent member 1 coupled and sutured with the proximal end of the proximal most stent-graft unit 2 and/or distal open stent member 7 coupled and sutured with the distal end of the distal most stent-graft unit 3.

Referring to the figures 3, the proximal stent-graft unit 2 and the distal most stent-graft unit 3 individually comprises a stent member 4 coupled and sutured with a tubular graft member 5 by the suture thread 6. The difference between the proximal stent-graft unit 2 and the distal most stent-graft unit 3 is that the distal most stent-graft unit 3 comprises a graft member 5 with a first end portion and a second end portion slightly exceeding the length of the stent member 4 while the proximal stent-graft units 2 have a graft member 5 with the first end portion slightly exceeding the length of the stent member 4 and the second end portion exceeding the length of the stent member 4 to a greater extent creating an overlapping length in the stent-graft unit 2.

While coupling to the tubular graft member 5 to make the proximal stent-graft unit 2 or the distal most stent-graft unit 3, the stent member 4 could either be disposed and sutured within the inner diameter or about the outer diameter of the graft member 5.

The axial length of the graft member of the distal most stent-graft unit 3 ranges from 10 to 40 mm, and the axial length of the graft member of proximal stent-graft units 2 range from 14 to 60 mm. Specially, the preferred axial length of the graft member of proximal stent-graft units 2 range from 25 to 40 mm, and the axial length of the graft member of the distal most stent-graft unit 3 ranges from 20 to 35 mm.

Referring to the figure 4, the stent-graft units 2 and 3 are interconnected and sutured together in adjacent turns with the proximal stent-graft unit 2 slip fit or disposed partially within the inner diameter of the distal stent-graft unit 2 or 3 and overlapping the adjoining distal stent-graft unit 2 or 3. While sutured together, each distal apex of the sine wave of stent member of the proximal stent-graft unit 2 is disposed circumferentially between the two proximal apexes of the sine wave of stent member of the distal stent-graft unit 2 or 3.

While coupling the proximal open frame stent member 1 to the proximal most stent-graft unit 2, the proximal open frame stent member 1 is disposed partially within the inner diameter of and overlapping the proximal end portion of the proximal most stent-graft unit 2, with each distal apex of the sine wave of the open frame stent member 1 disposed circumferentially between the two proximal apexes of the sine wave of the proximal most stent-graft unit 2.

While coupling the distal open frame stent member 7 to the distal most stent-graft unit 3, the distal open frame stent member 7 is disposed partially within the inner diameter of and overlapping the distal end portion of the distal most stent-graft unit 3, with each proximal apex of the sine wave of the open frame stent member 7 disposed circumferentially between the two proximal apexes of the sine wave of the distal most stent-graft unit 3.

Referring to the figure 1, the stent member is formed from a single continuous wire arranged in a complanate configuration with multiple bends and having multiple undulations with each undulation having an apex by cold deformation, or heat treatment, or combination of cold deformation and heat treatment, and then has two ends joined together arranged in a tubular configuration by welding the two ends directly, or welding or mechanical crimping using an alloy tubing.

Typically, each stent member 4 has 4 to 10 apexes or crowns among forming a frame loop. Preferably 4 to 6 crowns are in a frame loop. The longitudinal length of a stent member 4 ranges from approximately 10 mm to approximately 40 mm, but preferably 20 mm to 35 mm.

The total covered length of the multi-unit stent-graft without considering the length of proximal and distal open frame stent members 1 and 7 varies according application by changing the number of proximal stent-graft units 2.

Referring to the figure 1 and 2, the inner and outer diameter of the tubular multi-unit stent-graft 1 can be the same throughout or vary wherein the proximal inner or outer diameter and the distal inner or outer diameter are different forming a taper.

### Materials applied

Said alloy wire forming the stent members 4 should be suitable material with good biocompatibility, chemical stability and mechanical properties which satisfied for use in implantation in human body, such as nitinol (in either super-elastic or shape memory form), CoCr alloy, or 316L/317L stainless steel. The wire OD ranges from 0.254 to 0.635 mm.

The tubular graft members 2, 3 may manufactured from polytetrafluoroethylene (PTFE), expanded polytetrafluoroethylene (PTFE), polyester fabric, polyamide fabric, polypropylene, or other suitable biocompatible materials.

The suture thread may be made of any suitable material, such as polypropylene, polyester, polyamide, silk, polytetrafluoroethylene (PTFE), polyfluoroethylene, or other suitable biocompatible materials.

The alloy tube may be made of any suitable material, such as nitinol, 316U317L stainless steel, or CoCr alloy.

## Claims

1. A tubular endoluminal prosthesis comprising: a plurality of stent-graft units placed in adjacent turns with a proximal stent-graft unit overlapping partly and coupled to the distal stent-graft unit, said stent-graft unit comprising a supporting stent member formed from a single continuous wire arranged in a tubular configuration with multiple bends and having multiple undulations coupled to a graft member defining a tube, **characterized in that**
each stent member is discrete and separate from the stent member of an adjacent stent-graft unit and each graft member is discrete and separate from the graft member of an adjacent stent-graft unit, and wherein each graft member is connected with sutures to an adjacent graft member.

2. The tubular endoluminal prosthesis of claim 1, wherein the graft member of the proximal stent-graft unit has longer longitudinal length than that of the distal most stent-graft unit, with the length of said graft member of the proximal stent-graft unit ranging from 14 to 60 mm, and the length of said graft member of the distal most stent-graft unit ranges from 10 to 40 mm.

3. The tubular endoluminal prosthesis of any of the preceding claims, wherein the stent member each has 4 to 10 apexes or crowns forming a frame loop and the longitudinal length ranges from 10 mm to 40 mm.

4. The tubular endoluminal prosthesis of any of the preceding claims, wherein the stent member is disposed and sutured within the inner diameter of the tubular graft member.

5. The tubular endoluminal prosthesis of any of the preceding claims, wherein multiple stent-graft units are interconnected and an intermediate stent-graft unit is disposed partially within the inner diameter of the distal stent-graft unit, with each distal apex of the sine wave of stent member of said intermediate stent-graft unit disposed circumferentially between the two proximal apexes of the sine wave of the stent member of the distal stent-graft unit.

6. The tubular endoluminal prosthesis of any of the preceding claims further comprising a proximal open-frame stent member with the same sine wave configuration as the stent member, the proximal open-frame stent member partially overlapping the proximal end portion of the proximal most stent-graft unit and where each distal apex of the sine wave of the proximal open frame stent member is disposed circumferentially between the two proximal apexes of the sine wave of stent member of the proximal most stent-graft unit.

7. The tubular endoluminal prosthesis of any of the preceding claims further comprising a distal open-frame stent member with the same sine wave configuration as the stent member, the distal open-frame stent member partially overlapping the distal end portion of the distal most stent-graft unit and where each distal apex of the sine wave of the distal open frame stent member is disposed circumferentially between the two proximal apexes of the sine wave of stent member of the distal most stent-graft unit.

8. The tubular endoluminal prosthesis of any of the preceding claims further comprising an alloy tube made from 316L stainless steel or CoCr alloy, joining together the two ends of the wire forming the stent member arranged in a tubular configuration.

9. The tubular endoluminal prosthesis of any of the preceding claims, wherein the alloy wire be made from one of super-elastic nitinol, shape memory nitinol, 316L stainless steel or CoCr alloy and the wire ranging from 0.254 to 0.635 mm; the tubular graft members made from one of polytetrafluoroethylene (PTFE), expanded polytetrafluoroethylene (ePTFE), polyester fabric, polyamide fabric, polypropylene; the suture thread be made from one of polypropylene, polyester, polyamide, silk, polytetrafluoroethylene (PTFE), polyfluoroethylene.

## Patentansprüche

1. Röhrenförmige, endoluminale Prothese umfassend mehrere, in Form aneinandergrenzender Windungen angeordnete Stent-Graft-Einheiten, wobei eine proximale Stent-Graft-Einheit mit der distalen Stent-Graft-Einheit teilweise überlappt und mit dieser verbunden ist, wobei die Stent-Graft-Einheit ein stützendes Stent-Element umfasst, welches aus einem einzelnen, durchgehenden Draht geformt ist, wobei der Draht in einer röhrenförmigen Struktur mit mehreren Krümmungen und mehreren Wellungen angeordnet und mit einem eine Röhre definierenden Graft-Element verbunden ist, **dadurch gekennzeichnet, dass** jedes Stent-Element einzeln und getrennt von dem Stent-Element einer benachbarten Stent-Graft-Einheit ausgebildet ist und jedes Graft-Element einzeln und getrennt von dem Graft-Element einer benachbarten Stent-Graft-Einheit ausgebildet ist und wobei jedes Graft-Element durch Nähte mit einem benachbarten Graft-Element verbunden ist.

2. Röhrenförmige, endoluminale Prothese nach Anspruch 1, wobei das Graft-Element der proximalen Stent-Graft-Einheit eine längere Ausdehnung in longitudinaler Richtung aufweist als das Graft-Element der distal endständigen Stent-Graft-Einheit, wobei die Länge des Graft-Elements der proximalen Stent-Graft-Einheit von 14 mm bis 60 mm beträgt und wobei die Länge des Graft-Elements der distal endständigen Stent-Graft-Einheit von 10 mm bis 40 mm beträgt.

3. Röhrenförmige, endoluminale Prothese nach einem der vorhergehenden Ansprüche, wobei jedes Stent-Element vier bis zehn Scheitelpunkte oder Kronen aufweist und einen Gerüstring ausbildet und die Ausdehnung in longitudinaler Richtung von 10 mm bis 40 mm beträgt.

4. Röhrenförmige, endoluminale Prothese nach einem der vorhergehenden Ansprüche, wobei das Stent-Element innerhalb des Innendurchmessers des röhrenförmigen Graft-Elements angeordnet und angenäht ist.

5. Röhrenförmige, endoluminale Prothese nach einem der vorhergehenden Ansprüche, wobei mehrere Stent-Graft-Einheiten miteinander verbunden sind und wobei eine dazwischenliegende Stent-Graft-Einheit teilweise innerhalb des Innendurchmessers der distalen Stent-Graft-Einheit angeordnet ist, wobei jeder distale Scheitelpunkt der Sinuskurve des Stent-Elements der dazwischenliegenden Stent-Graft-Einheit umlaufend zwischen zwei proximalen Scheitelpunkten der Sinuskurve des Stent-Elements der distalen Stent-Graft-Einheit angeordnet ist.

6. Röhrenförmige, endoluminale Prothese nach einem der vorhergehenden Ansprüche zusätzlich umfassend ein proximales, freistehendes Stent-Element mit derselben Sinuskurven-Struktur wie das Stent-Element, wobei das proximale freistehende Stent-Element teilweise mit dem proximalen Endabschnitt der proximal endständigen Stent-Graft-Einheit überlappt und wobei jeder distale Scheitelpunkt der Sinuskurve des proximalen freistehenden Stent-Elements umlaufend zwischen zwei proximalen Scheitelpunkten der Sinuskurve des Stent-Elements der proximal endständigen Stent-Graft-Einheit angeordnet ist.

7. Röhrenförmige, endoluminale Prothese nach einem der vorhergehenden Ansprüche zusätzlich umfassend ein distales freistehendes Stent-Element aufweisend die selbe Sinuskurven-Struktur wie das Stent-Element, wobei das distale freistehende Stent-Element teilweise mit dem distalen Endabschnitt der distal endständigen Stent-Graft-Einheit überlappt und wobei jeder distale Scheitelpunkt der Sinuskurve des distalen freistehenden Stent-Elements umlaufend zwischen zwei proximalen Scheitelpunkten der Sinuskurve des Stent-Elements der distal endständigen Stent-Graft-Einheit angeordnet ist.

8. Röhrenförmige, endoluminale Prothese nach einem der vorhergehenden Ansprüche zusätzlich umfassend ein legiertes Rohr bestehend aus 316L Edelstahl oder einer CoCr Legierung, wobei das Rohr die beiden Enden des Drahtes miteinander verbindet, welcher das röhrenförmig angeordnete Stent-Element bildet.

9. Röhrenförmige, endoluminale Prothese nach einem der vorhergehenden Ansprüche, wobei der legierte Draht aus superelastischem Nitinol, Shapememory Nitinol, 316L Edelstahl oder einer CoCr Legierung hergestellt ist und wobei der Draht von 0.254 mm bis 0.635 mm reicht, wobei die röhrenförmigen Graft-Elemente aus Polytetrafluoroethylen (PTFE), expanded Polytetrafluoroethylen (ePTFE), Polyestergewebe, Polyamidgewebe oder Polypropylen hergestellt sind und wobei der Nähfaden aus Polypropylen, Polyester, Polyamid, Seide, Polytetrafluoroethylen (PTFE) oder Polyfluoroethylen hergestellt ist.

## Revendications

1. Prothèse endoluminale tubulaire comprenant: une pluralité d'éléments de greffon d'endoprothèse placés en des tours adjacentes avec un élément de greffon d'endoprothèse proximal couplé à un élément de greffon d'endoprothèse distal en le recouvrant partiellement, ledit élément de greffon d'endoprothèse comprenant un organe d'endoprothèse de soutien formé à partir d'un seul fil continu arrangé en une configuration tubulaire présentant de multiple courbes et ayant de multiple ondulations couplé à un organe de greffe définissant un tube, **caractérisée en ce que** chaque organe d'endoprothèse est discret et séparé de l'organe d'endoprothèse de l'élément de greffon d'endoprothèse adjacent et chaque organe de greffe est discret et séparé de l'organe de greffe d'un élément de greffon d'endoprothèse adjacent, et **en ce que** chaque organe de greffe est relié par des sutures à un organe de greffe adjacent.

2. Prothèse endoluminale tubulaire selon la revendication 1, **caractérisée en ce que** l'organe de greffe de l'élément de greffon d'endoprothèse proximal à une longueur longitudinale plus grande que celle de l'élément de greffon d'endoprothèse le plus distal, avec la longueur dudit organe de greffe de l'élément de greffon d'endoprothèse proximal allant de 14 à 60 mm, et la longueur dudit organe de greffe de l'élément de greffon d'endoprothèse le plus distal qui se situe de 10 à 40 mm.

3. Prothèse endoluminale tubulaire selon l'une des revendications précédentes, **caractérisée en ce que** chaque organe d'endoprothèse possède 4 à 10 apices ou extrados formant une structure en boucle et la longueur longitudinale se situe de 10 mm à 40 mm.

4. Prothèse endoluminale tubulaire selon l'une des revendications précédentes, **caractérisée en ce que** l'organe d'endoprothèse est disposé et suturé sur le diamètre intérieur de l'organe de greffe tubulaire.

5. Prothèse endoluminale tubulaire selon l'une des revendications précédentes, **caractérisée en ce qu'**une pluralité d'éléments de greffon d'endoprothèse sont interconnectés et un élément de greffon d'endoprothèse intermédiaire est disposé partiellement sur le diamètre intérieur de l'élément de greffon d'endoprothèse distal, en ayant chaque apex distal d'une courbure sinusoïdale de l'organe d'endoprothèse dudit élément de greffon d'endoprothèse intermédiaire disposé de façon circonférentielle entre les deux apices proximaux de la courbure sinusoïdale de l'organe d'endoprothèse de l'élément de greffon d'endoprothèse distal.

6. Prothèse endoluminale tubulaire selon l'une des revendications précédentes comprenant en outre un organe d'endoprothèse proximal à structure ouverte ayant la même configuration de courbure sinusoïdale que l'organe de greffe, l'organe d'endoprothèse proximal à structure ouverte recouvrant partiellement la portion d'extrémité proximale de l'élément de greffon d'endoprothèse le plus proximal et où chaque apex distal de la courbure sinusoïdale de l'organe d'endoprothèse à structure ouverte proximale est disposé de façon circonférentielle entre les deux apices proximaux de l'organe d'endoprothèse de courbure sinusoïdale de l'élément de greffon d'endoprothèse le plus proximal.

7. Prothèse endoluminale tubulaire selon l'une des revendications précédentes comprenant en outre un organe d'endoprothèse distal à structure ouverte ayant la même configuration de courbure sinusoïdale que l'organe d'endoprothèse, l'organe d'endoprothèse distal à structure ouverte recouvrant partiellement la portion d'extrémité distale de l'élément de greffon d'endoprothèse le plus distal et où chaque apex distal de la courbure sinusoïdale de l'organe d'endoprothèse distal à structure ouverte est disposé de façon circonférentielle entre les deux apices proximaux de l'organe d'endoprothèse de courbure sinusoïdale de l'élément de greffon d'endoprothèse le plus distal.

8. Prothèse endoluminale tubulaire selon l'une des revendications précédentes comprenant en outre un tube d'un alliage fabriqué à partir d'acier inoxydable 316L ou d'un alliage de CoCr, joignant ensemble les deux extrémités du fil formant l'organe d'endoprothèse disposé dans une configuration tubulaire.

9. Prothèse endoluminale tubulaire selon l'une des revendications précédentes, **caractérisée en ce que** le fil en alliage est fabriquée à partir d'un nitinol super élastique, un nitinol à mémoire de forme, un acier inoxydable 316L ou d'un alliage de CoCr et **en ce que** le fil s'étendant de 0.254 à 0.635 mm; les organes de greffe tubulaires étant fabriqués à partir d'un polytétrafluoroéthylène (PTFE), d'un polytétrafluoroéthylène expansé (ePTFE), d'un tissu en polyester, d'un tissu en polyamide, de polypropylène; le fil de suture étant fabriqué à partir d'un polypropylène, d'un polyester, d'un polyamide, de la soie, d'un polytétrafluoroéthylène (PTFE), d'un polyfluoroéthylène.
